(19) Europäisches Patentamt
European Patent Office
Office européen des brevets

(11) Veröffentlichungsnummer: **0 069 065**
**B1**

# (12) EUROPÄISCHE PATENTSCHRIFT

(45) Veröffentlichungstag der Patentschrift: **13.02.85**

(51) Int. Cl.⁴: **C 07 C 85/24**

(21) Anmeldenummer: **82810254.1**

(22) Anmeldetag: **14.06.82**

(54) **Verfahren zum Alkylieren oder Aralkylieren von aromatischen Aminen.**

(30) Priorität: **19.06.81 GB 8119010**

(43) Veröffentlichungstag der Anmeldung: **05.01.83 Patentblatt 83/1**

(45) Bekanntmachung des Hinweises auf die Patenterteilung: **13.02.85 Patentblatt 85/7**

(84) Benannte Vertragsstaaten: **BE CH DE FR GB LI**

(56) Entgegenhaltungen:
**GB - A - 468 226**
**US - A - 2 285 243**

**G.A. OLAH: "FRIEDEL-CRAFTS AND RELATED REACTIONS", part I, 1964, Interscience Publishers, New York, Londres, Sydney "II. Alkylation and related reactions", Seite 577**
**J. CHEM. SOC., 1937, Londres (GB) W.J. HICKINBOTTOM: "Rearrangement of the alkylanilines. Part VII. The behaviour of alkylanilines with tert.-alkyl groups", Seiten 404-406**

(73) Patentinhaber: **CIBA-GEIGY AG, Postfach, CH-4002 Basel (CH)**

(72) Erfinder: **Howell, Frederick Harold, Dr., 27 High Bank, Atherton Lancashire M29 9HZ (GB)**

**EP 0 069 065 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zum Alkylieren oder Aralkylieren von aromatischen Aminen und die so hergestellten aromatischen Amine.

Es ist bekannt, daß Anilin bei Temperaturen von im allgemeinen über 200°C (GB-PS 756 538) oder auch bei niedrigen Temperaturen alkyliert werden kann, sofern besonders reaktive Olefine eingesetzt und das gebildete Wasser laufend und vollständig entfernt werden, (GB-PS 989 584).

Aufgabe der Erfindung ist die Bereitstellung eines neuen Verfahrens zur Herstellung von in ortho- oder para-Stellung alkylierten oder aralkylierten Anilinen, bei dem weder hohe Temperaturen noch besonders reaktive Olefine benötigt werden, bei dem jedoch eine große Anzahl verschiedener Alkylierungsmittel oder Aralkylierungsmittel unter milderen Bedingungen als den bisher verwendeten eingesetzt werden können.

Es wurde nun gefunden, daß diese Aufgabe unter Vermeidung der beschränkten Anwendbarkeit der bekannten Verfahren überraschenderweise vollständig gelöst werden kann, indem man aromatische Amine in Gegenwart einer Säure als Katalysator in wäßrigem saurem Medium mit einem Alkylierungsmittel oder Aralkylierungsmittel umsetzt.

Gegenstand der Erfindung ist ein Verfahren zur Herstellung von Verbindungen der Formel I

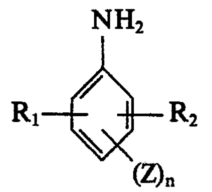

(I)

und Salzen oder Metallsalz-Komplexen davon mit organischen oder anorganischen Säuren, wobei n 1,2 oder 3, $R_1$ Wasserstoff, Halogen, $-CF_3$, $-CCl_3$, geradkettiges oder verzweigtes $C_{1-5}$-Alkyl oder Phenyl und $R_2$ Wasserstoff, Halogen oder geradkettiges oder verzweigtes $C_{1-5}$-Alkyl darstellen, Z in ortho- oder para-Stellung zur Aminogruppe gebunden ist und einen Rest der Formel

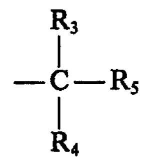

darstellt, worin $R_3$ Wasserstoff oder geradkettiges $C_{1-4}$-Alkyl, $R_4$ geradkettiges $C_{1-4}$-Alkyl oder Phenyl und $R_5$ geradkettiges oder verzweigtes $C_{1-8}$-Alkyl oder Phenyl oder $R_3$ und $R_4$ und/oder $R_4$ und $R_5$ zusammen mit dem C-Atom, an das sie gebunden sind, einen Cyclohexylenring bedeuten, indem man ein aromatisches Amin der Formel II

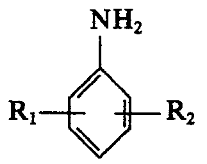

(II)

worin $R_1$ und $R_2$ die oben angegebene Bedeutung haben, bei erhöhter Temperatur in Gegenwart einer Säure als Katalysator in wäßrigem saurem Medium, das mindestens 30 Gew.-% Wasser, bezogen auf die eingesetzte Säure, enthält, mit bis zu drei Mol eines Alkylierungsmittels (III) oder Aralkylierungsmittels umsetzt, das zur Einführung eines Restes Z in den Benzolring des Amins der Formel II in ortho- oder para-Stellung zur Aminogruppe befähigt ist, und die Verbindungen der Formel I gegebenenfalls mit einem organischen oder anorganischen Salz in ein Salz oder einen Metallsalz-Komplex überführt.

Das Mol-Verhältnis von Amin der Formel II zu Alkylierungsmittel oder Aralkylierungsmittel (III) kann im Bereich von 10 : 1 bis 1 : 3 variieren. Wird das Amin der Formel II im Überschuß eingesetzt, so kann dieser Überschuß z. B. durch Destillation zurückgewonnen und wieder verwendet werden.

Stellen $R_1$ oder $R_2$ Halogen dar, so handelt es sich z. B. um F, Cl, Br oder J, bevorzugt F oder Cl. Beispiele von $C_{1-5}$-Alkylgruppen $R_1$ und $R_2$ sind die Methyl-, Äthyl-, n-Propyl-, Isopropyl-, n-Butyl-, sek.-Butyl-, Isobutyl-, tert.-Butyl-, n-Pentyl- oder Neopentylgruppe. Bevorzugte Alkylgruppen $R_1$ und $R_2$ sind Methyl und Äthyl.

Stellen $R_3$ und $R_4$ $C_{1-4}$-Alkylgruppen dar, so handelt es sich z. B. um Methyl-, Äthyl-, n-Propyl- oder n-Butylgruppen.

Bedeutet $R_5$ eine geradkettige oder verzweigte $C_{1-8}$-Alkylgruppe, so handelt es sich z. B. um eine Methyl- Äthyl-, n-Propyl-, Isopropyl-, n-Butyl-, sek.-Butyl-, Isobutyl-, n-Pentyl-, Neopentyl-, n-Hexyl-, n-Heptyl- oder n-Octylgruppe. Bilden $R_3$ und $R_4$ oder $R_4$ und $R_5$ zusammen mit dem C-Atom, an das sie

gebunden sind, einen Cyclohexylenrest, so stellt Z z. B. 1-Methyl-cyclohex-1-yl, 1-Äthyl-cyclohex-1-yl oder 1-Phenyl-cyclohex-1-yl dar.

Stellen $R_3$ und $R_4$ zusammen mit dem C-Atom, an das sie gebunden sind, einen Cyclohexylenrest dar und ist $R_5$ zusammen mit dem C-Atom, an das es gebunden ist, mit diesem Cyclohexylenrest so verbunden, daß ein weiterer Cyclohexylenrest gebildet wird, so kann Z eine Adamant-1-yl-gruppe darstellen.

Bevorzugte nach dem erfindungsgemäßen Verfahren hergestellte Verbindungen der Formel I sind solche, worin n und $R_1$ die oben angegebene Bedeutung haben, $R_2$ Wasserstoff, $R_3$ Wasserstoff oder Methyl, $R_4$ Methyl und $R_5$ $C_{1-8}$-Alkyl oder Phenyl bedeuten. Besonders bevorzugt sind nach dem erfindungsgemäßen Verfahren hergestellte Verbindungen der Formel I, worin n die oben angegebene Bedeutung hat, $R_1$ Wasserstoff oder Halogen, bevorzugt Cl, $R_2$ Wasserstoff, $R_3$ Wasserstoff oder Methyl, $R_4$ Methyl und $R_5$ $C_{1-8}$-Alkyl oder Phenyl darstellen.

Als Beispiele von Salzen von Verbindungen der Formel I seien genannt: Hydrochloride, Hydrobromide, Sulfate, Phosphate, Methansulfonate, p-Toluolsulfonate, Formiate, Oxalate, Adipate und Isophthalate. Als Metallsalz-Komplexe kommen beispielsweise Komplexe mit Zinkchlorid oder Zinn(II)chlorid in Betracht.

Beispiele von nach dem erfindungsgemäßen Verfahren hergestellten Verbindungen der Formel I sind:

4-tert.-Butylanilin, 4-(1,1,3,3-Tetramethyl-butyl)-anilin, 4-(Adamant-1-yl)-anilin, 4-Cumylanilin, 2-Cumylanilin, 2-Isopropylanilin, 4-Isopropylanilin, 2,4-Di-isopropylanilin, 2,6-Di-isopropylanilin, 2,4,6-Tri-isopropylanilin, 4-Cyclohexylanilin, 4-(1-Methyl-cyclohex-1-yl)-anilin, 4-tert.-Butyl-2-methylanilin, 4-Cumyl-2-methylanilin, 2-Cumyl-5-methylanilin, 2-Cumyl-4-methylanilin, 4-Cumyl-2,6-dimethylanilin, 4-Cumyl-2-äthylanilin, 4-Cumyl-2,6-diäthylanilin, 4-Cumyl-2-isopropylanilin, 4-Cumyl-2,6-diisopropylanilin, 4-Cumyl-2-sek.-butylanilin, 4-Cumyl-2-äthyl-6-methylanilin, 4-Cumyl-2-isopropyl-6-methylanilin, 4-Cumyl-2-sek.-butyl-6-methylanilin, 4-Cumyl-2-sek.-butyl-6-äthylanilin, 4-Cumyl-2-phenylanilin, 2-Chlor-4-tert.-butylanilin, 4-(Adamant-1-yl)-2-chloranilin, 2-Chlor-4-cumylanilin, 2-Chlor-4-cumyl-5-methylanilin, 4-tert.-Butyl-2,6-dichloranilin, 4-(Adamant-1-yl)-2,6-dichloranilin, 4-Cumyl-2,6-dichloranilin, 4-Cumyl-2,3-dichloranilin, 4-Cumyl-2,5-dichloranilin, 2-Brom-4-cumylanilin, 4-Cumyl-2-fluoranilin, 4-Cumyl-3-fluoranilin, 4-Cumyl-2-trifluormethylanilin und 4-Cumyl-2-trichlormethylanilin.

Die Umsetzung der aromatischen Amine der Formel II mit den Alkylierungsmitteln oder Aralkylierungsmittel wird bevorzugt in Gegenwart eines Metallsalzes als Co-Katalysator und gegebenenfalls bei Überdruck durchgeführt.

Alkylierungsmittel oder Aralkylierungsmittel (III) zur Umsetzung mit den Aminen der Formel II enthalten ein reaktives Zentrum, z. B. eine olefinische, Hydroxy-, Amino- oder Äthergruppe oder Halogen, das während der Reaktion eliminiert, umgewandelt oder umgelagert wird. Es wird angenommen, daß die Reaktion über einen Carbonium-Ion-Mechanismus verläuft; deswegen werden Alkylierungsmittel oder Alkarylierungsmittel, die tertiäre Carboniumionen liefern, vor solchen bevorzugt, die sekundäre Carboniumionen bilden, und die letzteren werden ihrerseits gegenüber Alkylierungsmitteln oder Alkarylierungsmitteln bevorzugt, die primäre Carboniumionen liefern.

Ein wesentliches Merkmal des erfindungsgemäßen Verfahrens ist das Vorhandensein von mindestens 30 Gew.-% Wasser im Reaktionsgemisch (bezogen auf die eingesetzte Säure). Das Vorliegen dieser wesentlichen Menge Wasser, bezogen auf die Säure, ist notwendig um sicherzustellen, daß das Reaktionsgemisch eine homogene Lösung bildet. Bei der bevorzugten Verwendung von Chlorwasserstoffsäure als Säure beträgt die Wassermenge vorzugsweise 64 Gew.-%, bezogen auf HCl, d. h. es wird handelsübliche konzentrierte HCl (36%ig) verwendet. Im Interesse einer wirtschaftlichen Ausbeute aus einem bestimmten Reaktorvolumen müssen selbstverständlich große Überschüsse an Wasser, bezogen auf das gesamte Reaktionsgemisch, vermieden werden.

Gewünschtenfalls kann ein weiteres Lösungsmittel mitverwendet werden, unter der Voraussetzung, daß es während der Umsetzung inert bleibt.

Die Umsetzung wird bei erhöhter Temperatur durchgeführt, z. B. einer Temperatur zwischen 30 und 250° C, insbesondere zwischen 100 und 190° C. Bei Reaktionstemperaturen über 110° C kann Überdruck in geeigneten Druckapparaturen, z. B. zugeschmolzenen Glasreaktoren oder säurebeständigen Druckgefäßen, wie mit Tantal ausgekleidete Reaktoren, angewendet werden. Wird bei Überdruck gearbeitet, so ist ein Druck unter 0,987 kPa bevorzugt.

Ein weiteres wesentliches Merkmal des erfindungsgemäßen Verfahrens ist die Verwendung einer Säure als Katalysator. Die Säure kann anorganisch oder organisch oder ein teilweises Salz einer anorganischen oder organischen Säure sein. Als Beispiele solcher Säuren seien genannt: Chlorwasserstoffsäure, Schwefelsäure und Orthophosphorsäure; alkyl-, aryl- oder alkaryl-substituierte anorganische Säuren, z. B. Methan- oder Äthansulfonsäuren, Benzolsulfonsäure, p-Toluolsulfonsäure und Methanphosphonsäure; Dichloressigsäure, Trichloressigsäure oder Trifluoressigsäure. Das Verhältnis von Katalysator (Säure) zu aromatischem Amin liegt vorzugsweise im Bereich von 0,25 bis 1,5 Mol und

besonders 0,5 bis 1,0 Mol. Die bevorzugte Säure ist Chlorwasserstoffsäure.

Die Alkylierung bzw. Aralkylierung wird mit Vorteil in Gegenwart eines Metallsalzes, -oxids, -hydroxids oder -carbonats als Co-Katalysator durchgeführt, wobei als Metalle solche der Gruppen II, III oder VIII des Periodischen Systems in Betracht kommen. Bevorzugte derartige Metalle sind Mg, Cd, Zn, Al, Fe, Co und Ni.

Bevorzugte Metallsalze sind solche, die mit der für die Katalysierung der Reaktion verwendeten Säure das Ion teilen, wie z. B. Halogenide, Sulfate oder Phosphate. Wird als Co-Katalysator ein Metallcarbonat, -oxid oder -hydroxid verwendet, so sollte die Säure in ausreichendem Überschuß über die für die Katalysierung der Alkylierung benötigte Menge vorliegen, um das Metallsalz zu bilden.

Das Metallsalz liegt zweckmäßig in einer Konzentration von bis zu 1,0 Mol, besonders zwischen 0,25 und 0,5 Mol, pro Mol aromatisches Amin, vor. Das bevorzugt als Co-Katalysator eingesetzte Metallhalogenid ist $ZnCl_2$.

Nach Beendigung der Alkylierung bzw. Aralkylierung wird das alkylierte bzw. aralkylierte aromatische Amin durch Behandlung mit einer Base von der Säure oder dem Säure/Metallsalz-Komplex befreit. Geeignete Basen für diesen Zweck sind Alkalimetallhydroxide, -carbonate und -bicarbonate sowie Ammoniumhydroxid. Natrium- und Ammoniumhydroxid werden als Basen bevorzugt. Diese Basen sind insbesondere dann bevorzugt, wenn der Co-Katalysator $ZnCl_2$ ist; dabei werden die anfänglich ausgefällten basischen Zinksalze schnell wieder durch überschüssige Base gelöst, wodurch eine einfache Isolierung der alkylierten bzw. aralkylierten aromatischen Amine ermöglicht wird.

Beispiele von Aminen der Formel II sind: Anilin, o-, m- und p-Toluidin, o-Äthylanilin, o-Isopropylanilin, o-sek.-Butylanilin, o- und m-Fluoranilin, o-Chloranilin, o-Bromanilin, 2,6-Dimethylanilin, 2,6-Diäthylanilin, 2,6-Diisopropylanilin, 2-Äthyl-6-methylanilin, 2-Methyl-6-isopropylanilin, 2-sek.-Buthyl-6-methylanilin, 2-sek.-Butyl-6-äthylanilin, 2-Chlor-5-methylanilin, 2-Chlor-6-methylanilin, 5-Chlor-2-methylanilin, 2-Phenylanilin, 2,6-Dichloranilin, 2,3-Dichloranilin, 2,5-Dichloranilin, 2-Trifluormethylanilin und 2-Trichlormethylanilin.

Als Alkylierungsmittel oder Aralkylierungsmittel (III) können im erfindungsgemäßen Verfahren Olefine, Alkohole, Alkylamine, Alkylhalogenide oder Äther verwendet werden, die zur Einführung eines Restes Z in den Benzolkern des aromatischen Amins der Formel II befähigt sind. Das reaktive Zentrum in den Alkylierungsmitteln bzw. Aralkylierungsmitteln (III) kann Wasserstoff, OH, Halogen, eine olefinische Bindung oder eine $NH_2$-Gruppe an einem tertiären C-Atom sein. Als Beispiele von Alkylierungsmitteln bzw. Aralkylierungsmitteln (III) seien genannt:

a)Olefine

Die Olefine können geradkettig oder verzweigt, cyclisch oder durch Phenyl substituiert sein. Beispiele von Olefinen, die sich zur Alkylierung oder Aralkylierung von aromatischen Aminen eignen, sind: Propylen, Buten-1, Buten-2 (cis oder trans), Penten-1, Penten-2, (cis oder trans), Hexen-1, Hexen-2 (cis oder trans), Hepten-1, Octen-1, Isobutylen, m-Methylbuten-1, 2-Methylpenten-1, 2,4-Dimethylpenten-1, 2,5-Dimethylhexen-1, 2,4,4-Trimethylpenten-1 und 2,4,4-Trimethylpenten-2 (Diisobutylen), Cyclohexen, 1-Methylcyclohex-1-en, 1-Äthylcyclohex-1-en, 1-Phenylcyclohex-1-en, Styrol, $\alpha$-Methylstyrol.

b) Alkohole

Als Alkylierungsmittel bzw. Aralkylierungsmittel können auch Alkohole verwendet werden. Die Alkohole können geradkettig, verzweigt, cyclisch oder durch Phenyl substituiert sein. Es können primäre, sekundäre oder tertiäre Alkohole verwendet werden. Tertiäre Alkohole werden gegenüber sekundären bevorzugt, die ihrerseits gegenüber primären bevorzugt sind. Es ist zu beachten, daß gewisse primäre Alkohole jedoch unter den Reaktionsbedingungen die bevorzugteren sekundären oder tertiären Carboniumionen bilden können. Beispiele von für die Alkylierung von aromatischen Aminen geeigneten Alkohole sind: Isopropanol, n-Butanol, Isobutanol, sek.-Butanol, tert.-Butanol, 2-Methylbutan-2-ol, 1-Methylcyclohexan-1-ol, 1-Isopropylcyclohexan-2-ol, $\alpha,\alpha$-Dimethylbenzylalkohol, 2,5-Dimethylhexan-2-ol, 2,4-Dimethylpentan-2-ol und 1-Adamantanol.

c)Amine

Zur Alkylierung von aromatischen Aminen können auch durch tertiäres Alkyl substituierte Amine mit einer Aminogruppe an einem tertiären C-Atom verwendet werden, wie z. B. tert.-Butylamin.

d)Halogenide

Die Alkylhalogenide bzw. Aralkylhalogenide können primär, sekundär oder tertiär sein und eine

gerade oder verzweigte Kette aufweisen. Dabei kann es sich um Alkyl-, Cycloalkyl oder Phenylalkylhalogenide handeln. Als Beispiele solcher Verbindungen seien genannt: tert.-Butylchlorid, 2,4,4-Trimethyl-2-chlorpentan oder $\alpha,\alpha$-Dimethyl-benzylchlorid.

e) Äther

Als Äther kommen Dialkyl- oder Alkylphenyläther in Betracht. Beispiele solcher Verbindungen sind der $\alpha,\alpha$-Dimethylbenzylmethyläther und der Phenyl-tert.-butyläther.

Von den obigen Alkylierungsmitteln bzw. Aralkylierungsmittel werden Olefine und Alkohole bevorzugt. Im allgemeinen werden Alkylierungsmittel, die ein tertiäres Carbonium liefern, bevorzugt. Besonders bevorzugte Alkylierungsmittel sind Isobutylen, Diisobutylen, Propylen oder $\alpha$-Methylstyrol.

Das erfindungsgemäße Verfahren eignet sich zum Alkylieren oder Aralkylieren einer Vielzahl von im Kern durch Alkyl-, Phenyl- oder Halogenalkylgruppen oder Halogenatome substituierten aromatischen Aminen, ausgehend von Anilin selbst bis zu mehrfach substituierten Anilinen. Im Gegensatz dazu lassen sich mit vorbekannten Verfahren nur Aniline oder einfache Derivate davon alkylieren, z. B. p-Amino-phenol, o- oder p-Anisidin oder Ester der Anthranilsäure.

Im erfindungsgemäßen Verfahren kann eine große Zahl von Alkylierungsmitteln bzw. Aralkylierungsmitteln mit einem breiten Molekulargewichtsbereich, z. B. von $C_3$ bis $C_{16}$, einschließlich Olefinen, Alkoholen, Aminen, Halogeniden und Estern, verwendet werden. Im Vergleich zu vorbekannten Verfahren wird das erfindungsgemäße Verfahren unter milderen Reaktionsbedingungen (niedrigere Temperaturen und geringerer Druck) durchgeführt.

Die Verbindungen der Formel I stellen wertvolle Zwischenprodukte zur Herstellung von Farbstoffen, Zusätzen für Kunststoff (vgl. z. B. britische Patentschrift Nr. 1 347 008), Kautschuke, Öle und dergleichen sowie zur Herstellung von biologisch aktiven Substanzen dar (vgl. z. B. britische Patentschrift Nr. 1 219 698 und 1 250 224).

Die Erfindung wird in den folgenden Beispielen näher erläutert. Teile und Prozente sind Gewichtsteile bzw. Gewichtsprozente, falls nichts anderes angegeben ist. Der Druck ist in Millibar angegeben.

Beispiel 1

279 Teile Anilin, 222 Teile tert.-Butylalkohol und 153 Teile 36%ige wäßrige Chlorwasserstoffsäure, in der zuvor 102 Teile wasserfreies Zinkchlorid gelöst worden sind, werden in einen mit Tantal ausgekleideten 1-Liter Autoklaven gegeben und 90 Stunden bei 175°C gerührt. Nach dem Entfernen aus dem Autoklaven wird das abgekühlte Reaktionsgemisch mit 750 Teilen Natriumhydroxid in 1500 Teilen Wasser behandelt. Die organische Phase wird abgetrennt, mit Wasser gewaschen und destilliert. Man erhält 407 Teile einer Fraktion, die bei 70—150°C/20 Millibar siedet. Gemäß Ermittlung der Konzentration in Bodenhöhe [GLC = ground level concentration] enthält das Destillat folgende Komponenten: 7,2 Gew.-% Anilin, 82,0% Gew.-% p-tert.-Butylanilin [Sdp. nach Literatur 228—230°C, Smp. 17°C], 4,6 Gew.-% 2,4-Di-tert.-butylanilin. Die Ausbeute an p-tert.-Butylanilin beträgt 75%.

Beispiel 2

Das Verfahren gemäß Beispiel 1 wird wiederholt unter Verwendung von 336 Teilen Diisobutylen anstelle von tert.-Butylalkohol. Die Destillation ergibt 411 Teile einer bei 60—180°C/20 Millibar siedenden Fraktion, die gemäß Ermittlung der Konzentration in Bodenhöhe folgende Komponenten enthält: 21,9 Gew.-% Anilin. 2,5 Gew.-% p-tert.-Butylanilin, 71,4 Gew.-% p-(1,1,3,3-Tetramethylbutyl)-anilin, 4,2 Gew.-% nicht identifizierter Bestandteile. Die Ausbeute an p-(1,1,3,4-Tetramethylbutyl)anilin beträgt 48%. Durch fraktionierte Destillation der obigen Fraktion erhält man reines p-(1,1,3,3-Tetramethylbutyl)-anilin vom Smp. 153—4°C/20 Millibar (Literatur 112—115°C/7 Millibar) mit der folgenden Zusammensetzung

| | | | |
|---|---|---|---|
| gefunden | C 81,89 | H 11,29 | N 6,96% |
| berechnet für $C_{14}H_{23}N$ | C 81,89 | H 11,29 | N 6,82% |

Beispiel 3

14,0 Teile Anilin, 15,3 Teile 36%ige Chlorwasserstoffsäure, 10,2 Teile Zinkchlorid und 11,4 Teile 1-Adamantanol werden in ein Glasrohr eingeschmolzen und in einen Schüttel-Autoklaven gegeben, dessen Druck mit Stickstoff auf 0,1974 kPa gebracht wird. Nach dem Schütteln während 33 Stunden bei 175°C ergibt das Reaktionsprodukt nach der Zersetzung mit 50 Teilen Natriumhydroxid in 100 Teilen Wasser 13,3 Teile 4-(1-Adamantyl)-anilin; Sdp. 174—6°C/0,7 Millibar (78% d. Th.). Die Kristallisation aus Äthanol ergibt farblose Plättchen vom Smp. 106—8°C (nach Literatur 105°C) mit der folgenden Zusammensetzung:

| | | | |
|---|---|---|---|
| gefunden | C 84,30 | H 9,07 | N 6,08% |
| berechnet für $C_{16}H_{21}N$ | C 84,53 | H 9,31 | N 6,16% |

Beispiel 4

18,6 Teile Anilin, 20,4 Teile 36%ige Chlorwasserstoffsäure, die 13,6 Teile Zinkchlorid enthält, werden 24 Stunden unter Rückfluß mit 23,6 Teilen $\alpha$-Methylstyrol gerührt. Die Aufarbeitung erfolgt wie in Beispiel 3 angegeben und ergibt nach der Destillation 32,9 Teile 4-Cumylanilin, Sdp. 175—80°C/4 Millibar (78% Ausbeute, bezogen auf 93%ige Reinheit gemäß Konzentration in Bodenhöhe). Das Produkt wird durch Hydrolyse des entsprechenden N-Acetylderivats (Smp. 129—31°C) gereinigt. Das reine 4-Cumylanilin hat einen Siedepunkt von 166—8°C/4 Millibar und die folgende Zusammensetzung:

| | | | |
|---|---|---|---|
| gefunden | C 85,23 | H 8,19 | N 6,66% |
| berechnet für $C_{15}H_{17}N$ | C 85,26 | H 8,11 | N 6,63%. |

Beispiel 5

18,6 Teile Anilin, 38,0 Teile p-Toluolsulfonsäure-monohydrat, 25 Teile Wasser und 23,6 Teile $\alpha$-Methylstyrol werden 24 Stunden unter Rückfluß gerührt. Die nach dieser Zeit noch vorhandene organische Phase wird nach dem Verdünnen mit Diäthyläther entfernt, und die wäßrige Phase wird dann mit Natriumhydroxidlösung alkalisch gestellt. Die organische Phase wird mit Diäthyläther isoliert und ergibt 14,6 Teile Destillat mit Sdp. 186—214°C/20 Millibar. Gemäß Ermittlung der Konzentration in Bodenhöhe enthält das Destillat folgende Komponenten: 15,1 Gew.-% 2-Cumylanilin, 21,5 Gew.-% $\alpha$-Methylstyrol-Dimer, 60,2 Gew.-% 4-Cumylanilin.

Beispiel 6

9,3 Teile Anilin, 10,2 Teile 36%ige Chlorwasserstoffsäure, 6,8 Teile Zinkchlorid und 12,6 Teile Propylen werden in ein Glasrohr eingeschmolzen und in einen Schüttel-Autoklaven gegeben, dessen Druck mit Stickstoff auf 0,588 kPa gebracht wird. Nach dem Schütteln während 60 Stunden bei 175°C wird das Reaktionsprodukt mit 25 Teilen Natriumhydroxid in 50 Teilen Wasser behandelt und ergibt nach der Destillation 19,5 Teile einer bei 120—155°C/20 Millibar siedenden Fraktion. Gemäß Ermittlung der Konzentration in Bodenhöhe enthält das Destillat folgende Komponenten: 0,9 Gew.-% Anilin, 2,3 Gew.-% o-Isopropylanilin, 2,3 Gew.-% p-Isopropylanilin, 5,0 Gew.-% 2,6-Diisopropylanilin, 7,5 Gew.-% 2,4-Diisopropylanilin, 64,1 Gew.-% 2,4,6-Triisopropylanilin.

Nach den in den vorangehenden Beispielen beschriebenen Verfahren werden die in der Tabelle 1 angeführten Verbindungen der Beispiele 7—27 hergestellt.

Tabelle 1

| Beispiel Nr. | Ausgangsprodukte/ Reaktionsbedingungen | Produkt | Sdp. °C/ Millibar | Smp. °C | Molekular-formel | Analyse % (berechnet/gefunden) C | H | N |
|---|---|---|---|---|---|---|---|---|
| 7 | 21,4 T. o-Toluidin/ 23,6 T. $\alpha$-Methylstyrol, 17 Std. bei Rückfluß | 4-Cumyl-2-methylanilin | 207—210/20 | 54—55 | $C_{16}H_{19}N$ | 85,27<br>85,28 | 8,54<br>8,50 | 6,03<br>6,22 |
| 8 | 21,4 T. m-Toluidin/ 23,6 T. 2-Methylstyrol, 17 Std. bei Rückfluß | 2-Cumyl-5-methyl-anilin | 209—254/20 (gemeinsam) | HCl-Salz 100—110 | $C_{16}H_{20}ClN$ | 73,42<br>73,40 | 7,79<br>7,70 | 5,07<br>5,35 |
| | | 4-Cumyl-3-methylanilin | | Zers. 71—3 | $C_{16}H_{19}N$ | 85,59<br>85,28 | 8,25<br>8,50 | 5,96<br>6,22 |
| | | 4,6-Dicumyl-3-methylanilin | | 184—186 | $C_{25}H_{29}N$ | 87,55<br>87,41 | 8,80<br>8,51 | 3,90<br>4,08 |
| 9 | 21,4 T. p-Toluidin/ 23,6 T. $\alpha$-Methylstyrol, 17 Std. bei Rückfluß | 2-Cumyl-4-methylanilin | 192—197/20 | 69—71 | $C_{16}H_{19}N$ | 84,49<br>85,28 | 8,45<br>8,50 | 6,48<br>6,22 |
| 10 | 24,2 T. 2,6-Dimethylanilin/ 23,6 T. $\alpha$-Methylstyrol, 6 Std. bei Rückfluß | 4-Cumyl-2,6-dimethylanilin | 142—147/0,3 | | $C_{17}H_{21}N$ | 85,59<br>85,30 | 8,82<br>8,85 | 5,70<br>5,85 |
| 11 | 18,6 T. Anilin/ 18,4 T. Benzhydrol 3 Std. bei Rückfluß | 2-(Diphenylmethyl)-anilin | 178—186/0,3 (gemeinsam) | 126 | $C_{19}H_{17}N$ | 87,68<br>87,99 | 6,62<br>6,61 | 4,94<br>5,40 |
| | | 4-(Diphenylmethyl)-anilin | | 85—87 | $C_{19}H_{17}N$ | 87,98<br>87,99 | 6,75<br>6,61 | 5,32<br>5,40 |
| | | 2,4-Bis-(diphenylmethyl)-anilin | | 118—120 | $C_{32}H_{27}N$ | 90,17<br>90,31 | 6,33<br>6,40 | 2,91<br>3,29 |

Tabelle 1

| Beispiel Nr. | Ausgangsprodukte/ Reaktionsbedingungen | Produkt | Sdp. °C/ Millibar | Smp. °C | Molekular-formel | Analyse % (berechnet/gefunden) C | H | N |
|---|---|---|---|---|---|---|---|---|
| 7 | 21,4 T. o-Toluidin/ 23,6 T. α-Methylstyrol, 17 Std. bei Rückfluß | 4-Cumyl-2-methylanilin | 207—210/20 | 54—55 | $C_{16}H_{19}N$ | 85,27<br>85,28 | 8,54<br>8,50 | 6,03<br>6,22 |
| 8 | 21,4 T. m-Toluidin/ 23,6 T. 2-Methylstyrol, 17 Std. bei Rückfluß | 2-Cumyl-5-methyl-anilin | 209—254/20 (für beide Produkte) | HCl-Salz 100—110 | $C_{16}H_{20}ClN$ | 73,42<br>73,40 | 7,79<br>7,70 | 5,07<br>5,35 |
| | | 4-Cumyl-3-methylanilin | | Zers. 71—3 | $C_{16}H_{19}N$ | 85,59<br>85,28 | 8,25<br>8,50 | 5,96<br>6,22 |
| | | 4,6-Dicumyl-3-methylanilin | | 184—186 | $C_{25}H_{29}N$ | 87,55<br>87,41 | 8,80<br>8,51 | 3,90<br>4,08 |
| 9 | 21,4 T. p-Toluidin/ 23,6 T. α-Methylstyrol, 17 Std. bei Rückfluß | 2-Cumyl-4-methylanilin | 192—197/20 | 69—71 | $C_{16}H_{19}N$ | 84,49<br>85,28 | 8,45<br>8,50 | 6,48<br>6,22 |
| 10 | 24,2 T. 2,6-Dimethylanilin/ 23,6 T. α-Methylstyrol, 6 Std. bei Rückfluß | 4-Cumyl-2,6-dimethylanilin | 142—147/0,3 | | $C_{17}H_{21}N$ | 85,59<br>85,30 | 8,82<br>8,85 | 5,70<br>5,85 |
| 11 | 18,6 T. Anilin/ 18,4 T. Benzhydrol 3 Std. bei Rückfluß | 2-(Diphenylmethyl)-anilin | 178—186/0,3 (für beide Produkte) | 126 | $C_{19}H_{17}N$ | 87,68<br>87,99 | 6,62<br>6,61 | 4,94<br>5,40 |
| | | 4-(Diphenylmethyl)-anilin | | 85—87 | $C_{19}H_{17}N$ | 87,98<br>87,99 | 6,75<br>6,61 | 5,32<br>5,40 |
| | | 2,4-Bis-(diphenylmethyl)-anilin | | 118—120 | $C_{32}H_{27}N$ | 90,17<br>90,31 | 6,33<br>6,40 | 2,91<br>3,29 |

Fortsetzung

| Beispiel Nr. | Ausgangsprodukte/ Reaktionsbedingungen | Produkt | Sdp. °C/ Millibar | Smp. °C | Molekular-formel | Analyse % (berechnet/gefunden) C | H | N |
|---|---|---|---|---|---|---|---|---|
| 19 | 32,4 T. 2,6-Dichloranilin/ 22,4 T. Diisobutylen, 60 Std. bei 125°C *) 1) | 4-tert.-Butyl-2,6-dichloranilin | 144/20 | | $C_{10}H_{13}Cl_2N$ | 55,01 55,06 | 5,98 6,01 | 6,70 6,42 |
| 20 | 16,2 T. 2,6-Dichloranilin/ 7,8 T. 1-Adamantanol, 33 Std. bei 150°C *) 2) | 4-(1-Adamantyl)-2,6-dichloranilin | 188—190/0,5 | 132—123 | $C_{16}H_{19}Cl_2N$ | 65,24 64,87 | 6,72 6,46 | 4,65 4,73 |
| 21 | 32,4 T. 2,6-Dichloranilin/ 23,6 T. $\alpha$-Methylstyrol, 24 Std. bei Rückfluß | 4-Cumyl-2,6-dichloranilin | | 92—94 | $C_{15}H_{15}Cl_2N$ | 64,54 64,29 | 5,64 5,40 | 5,26 5,00 |
| 22 | 32,4 T. 2,3-Dichloranilin/ 23,6 T. $\alpha$-Methylstyrol, 48 Std. bei Rückfluß | 4-Cumyl-2,3-dichloranilin | 140—180/0,4 | N-Acetyl 134—136 | $C_{17}H_{17}Cl_2N$ | 63,20 63,36 | 5,18 5,32 | 4,55 4,35 |
| 23 | 32,4 T. 2,5-Dichloranilin/ 23,6 T. $\alpha$-Methylstyrol, 24 Std. bei Rückfluß | 4-Cumyl-2,5-dichloranilin | 140—165/0,5 | 65—67 | $C_{15}H_{15}Cl_2N$ | 64,31 64,29 | 5,59 5,40 | 4,73 5,00 |
| 24 | 34,4 T. 2-Bromanilin/ 23,6 T. $\alpha$-Methylstyrol, 5 Std. bei Rückfluß | 2-Brom-4-cumylanilin | 162—170/0,5 | | $C_{15}H_{16}BrN$ | 62,05 62,08 | 5,56 5,56 | 4,60 4,83 |

*) In Glasrohr eingeschmolzen.
1) Unter Zusatz von 20 Teilen Wasser.
2) Unter Zusatz von 10 Teilen Wasser, 10,2 Teilen 36%iger wäßriger HCl und 6,8 Teilen $ZnCl_2$.

Fortsetzung

| Beispiel Nr. | Ausgangsprodukte/ Reaktionsbedingungen | Produkt | Sdp. °C/ Millibar | Smp. °C | Molekular-formel | Analyse % (berechnet/gefunden) C | H | N |
|---|---|---|---|---|---|---|---|---|
| 25 | 22,2 T. o-Fluoranilin/ 23,6 T. α-Methylstyrol, 11 Std. bei Rückfluß | 4-Cumyl-2-fluoranilin | 138—152/0,8 | 51—52 | $C_{15}H_{16}FN$ | 78,32<br>78,57 | 6,92<br>7,04 | 6,36<br>6,11 |
| 26 | 22,2 T. m-Fluoranilin/ 23,6 T. α-Methylstyrol, 3 Std. bei Rückfluß | 4-Cumyl-3-fluoranilin | 140—159/0,8 | 79—81 | $C_{15}H_{16}FN$ | 78,75<br>78,57 | 6,97<br>7,04 | 5,98<br>6,11 |
| 27 | 32,2 T. Trifluormethylanilin/ 23,6 T. α-Methylstyrol, 1 Std. bei Rückfluß | 4-Cumyl-2-trifluormethylanilin | 178—180/20 | 44—47 | $C_{16}H_{16}F_3N$ | 68,76<br>68,80 | 5,83<br>5,78 | 5,10<br>5,02 |

Alle Versuche unter Verwendung von 20,4 Teilen 36%iger wäßriger HCl und 13,6 Teilen $ZnCl_2$, außer wo anders angegeben.

**Patentansprüche**

1. Verfahren zur Herstellung von Verbindungen der Formel I

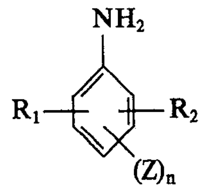

(I)

und Salzen oder Metallsalz-Komplexen davon mit organischen oder anorganischen Säuren, wobei n 1, 2 oder 3, $R_1$ Wasserstoff, Halogen, $-CF_3$, $-CCl_3$, geradkettiges oder verzweigtes $C_{1-5}$-Alkyl oder Phenyl und $R_2$ Wasserstoff, Halogen oder geradkettiges oder verzweigtes $C_{1-5}$-Alkyl bedeuten, Z in ortho- oder para-Stellung zur Aminogruppe gebunden ist und einen Rest der Formel

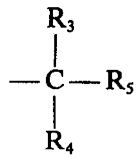

darstellt, worin $R_3$ Wasserstoff oder geradkettiges $C_{1-4}$-Alkyl, $R_4$ geradkettiges $C_{1-4}$-Alkyl oder Phenyl und $R_5$ geradkettiges oder verzweigtes $C_{1-8}$-Alkyl oder Phenyl oder $R_3$ und $R_4$ und/oder $R_4$ und $R_5$ zusammen mit dem C-Atom, an das sie gebunden sind, einen Cyclohexylenring bedeuten, dadurch gekennzeichnet, daß man ein aromatisches Amin der Formel II

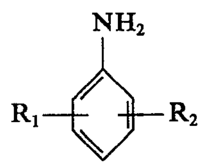

(II)

worin $R_1$ und $R_2$ die unter Formel I angegebene Bedeutung haben, bei erhöhter Temperatur in Gegenwart einer Säure als Katalysator in wäßrigem saurem Medium, das mindestens 30 Gew.-% Wasser, bezogen auf die eingesetzte Säure, enthält, mit bis zu drei Mol eines Alkylierungsmittels oder Aralkylierungsmittels (III) umsetzt, das zur Einführung eines Restes Z in den Benzolring des Amins der Formel II in ortho- oder para-Stellung zur Aminogruppe befähigt ist, und die Verbindungen der Formel I gegebenenfalls mit einem organischen oder anorganischen Salz in ein Salz oder einen Metallsalz-Komplex überführt.

2. Verfahren nach Anspruch 1, worin das Mol-Verhältnis von Amin der Formel II zu Alkylierungsmittel oder Aralkylierungsmittel (III) zwischen 10 : 1 bis 1 : 3 liegt.

3. Verfahren nach Anspruch 1, worin n und $R_1$ die im Anspruch 1 angegebene Bedeutung haben, $R_2$ Wasserstoff, $R_3$ Wasserstoff oder Methyl, $R_4$ Methyl und $R_5$ $C_{1-8}$-Alkyl oder Phenyl bedeuten.

4. Verfahren nach Anspruch 1, worin n die im Anspruch 1 angegebene Bedeutung hat, $R_1$ Wasserstoff oder Halogen, $R_2$ Wasserstoff, $R_3$ Wasserstoff oder Methyl, $R_4$ Methyl und $R_5$ $C_{1-8}$-Alkyl oder Phenyl bedeuten.

5. Verfahren nach Anspruch 1, dadurch gekennzeichnet, daß man die Umsetzung bei einer Temperatur zwischen 30 und 250° C durchführt.

6. Verfahren nach Anspruch 1, dadurch gekennzeichnet, daß man die Umsetzung bei einer Temperatur zwischen 100 und 190° C durchführt.

7. Verfahren nach Anspruch 1, dadurch gekennzeichnet, daß man die Umsetzung bei einer Temperatur über 110° C und einem Überdruck unter 0,9 87 kPa durchführt.

8. Verfahren nach Anspruch 1, dadurch gekennzeichnet, daß man als Katalysator Chlorwasserstoff verwendet.

9. Verfahren nach Anspruch 1, dadurch gekennzeichnet, daß man die Umsetzung in Gegenwart eines Metallsalzes, -oxids, -hydroxids oder -carbonats als Co-Katalysator durchführt, wobei das Metall ein solches der Gruppen II, III oder VIII des periodischen Systems ist.

10. Verfahren nach Anspruch 9, dadurch gekennzeichnet, daß das Metallsalz in einer Konzentration von bis zu 1,0 Mol pro Mol aromatisches Amin vorliegt.

11. Verfahren nach Anspruch 9, worin der Co-Katalysator $ZnCl_2$ ist.

12. Verfahren nach Anspruch 1, dadurch gekennzeichnet, daß man als Alkylierungsmittel oder Aralkylierungsmittel (III) Olefine, Alkohole, Alkylamine, Alkylhalogenide oder Äther verwendet, die zur Einführung des Restes Z in den Benzolring des aromatischen Amins der Formel II befähigt sind.

13. Verfahren nach Anspruch 1, dadurch gekennzeichnet, daß man als Alkylierungsmittel Isobutylen, Diisobutylen, Propylen oder α-Methylstyrol verwendet.

**Claims**

1. A process for producing a compound having the formula I

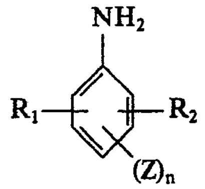

(I)

as well as salts or metal salt complexes thereof with organic or inorganic acids, in which formula I n is 1, 2 or 3, $R_1$ is H, halogen, $-CF_3$, $-CCl_3$, $C_1-C_5$ straight or branch chain alkyl or phenyl, $R_2$ is H, halogen, or $C_1-C_5$ straight or branch chain alkyl, and Z is linked at a position ortho or para to the $NH_2$ group and is a residue of formula

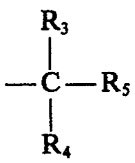

wherein $R_3$ is H or $C_1-C_4$ straight chain alkyl, $R_4$ is $C_1-C_4$ straight chain alkyl or phenyl and $R_5$ is $C_1-C_8$ straight or branch chain alkyl or phenyl, or $R_3$ and $R_4$ and/or $R_4$ and $R_5$, together with the carbon atom to which they are bonded, may be a cyclohexylene ring, which comprises reacting an aromatic amine having the formula II

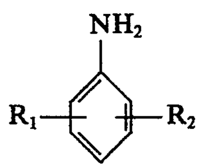

(II)

wherein $R_1$ and $R_2$ are as defined in formula I, with up to three moles of an alkylating or aralkylating agent (III) capable of introducing a residue Z into the benzene ring of an amine of formula II at a position ortho or para to the $NH_2$ group, the reaction being effected at elevated temperature in the presence of an acid catalyst in an aqueous acid medium containing at least 30% by weight of water, based on the acid used, and optionally converting the compound of formula I into a salt, or metal salt complex, with an organic or inorganic salt.

2. A process according to claim 1 wherein the molar proportion of amine of formula II to alkylating or aralkylating agent III is from 10 : 1 to 1 : 3.

3. A process according to claim 1 wherein, in the compound of formula I, n and $R_1$ are as defined in claim 1, $R_2$ is H, $R_3$ is H or $CH_3$, $R_4$ is $CH_3$ and $R_5$ is $C_1-C_8$ alkyl or phenyl.

4. A process according to claim 1 wherein, in the compound of formula I, n is as defined in claim 1, $R_1$ is H or halogen, $R_2$ is H, $R_3$ is H or $CH_3$, $R_4$ is $CH_3$ and $R_5$ is $C_1-C_8$ alkyl or phenyl.

5. A process according to claim 1 wherein the reaction is effected at a temperature in the range from 30° to 250° C.

6. A process according to claim 1 wherein the reaction is effected at a temperature in the range from 100° to 190° C.

7. A process according to claim 1 wherein the reaction temperature is above 110° C and a superatmospheric pressure of less than 0,987 kPa is applied.

8. A process according to claim 1 wherein the catalyst is hydrochloric acid.

9. A process according to claim 1 wherein the reaction is effected in the presence of a co-catalyst which is a metal salt, oxide, hydroxide or carbonate of a metal of Group II, III or VIII of the Periodic Table of Elements.

10. A process according to claim 9 wherein the metal salt is present in a concentration of up to 1 mole per mole of aromatic amine.

11. A process according to claim 9 wherein the co-catalyst is $ZnCl_2$.

12. A process according to claim 1 wherein the alkylating or aralkylating agent III is an olefin, an alcohol, an alkylamine, an alkyl halide or ether which is capable of introducing a residue Z into the benzene nucleus of the aromatic amine of formula II.

13. A process according to claim 1 wherein the alkylating agent is isobutylene, diisobutylene,

propylene or α-methylstyrene.

**Revendications**

1. Procédé de préparation de composés répondant à la formule (I):

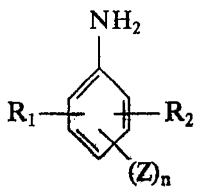

(I)

dans laquelle:

n représente un nombre égal à 1, à 2 ou à 3,
$R_1$ représente l'hydrogène, un halogène, $-CF_3$, $-CCl_3$, un alkyle en $C_1-C_5$ linéaire ou ramifié ou un phényle,
$R_2$ représente l'hydrogène, un halogène ou un alkyle en $C_1-C_5$ linéaire ou ramifié, et
Z est en ortho ou en para relativement au radical amino et représente un radical:

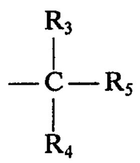

dans laquel $R_3$ représente l'hydrogène ou un alkyle linéaire en $C_1-C_4$, $R_4$ représente un alkyle linéaire en $C_1-C_4$ ou un phényle et $R_5$ représente un alkyle en $C_1-C_8$, linéaire ou ramifié, ou un phényle, ou encore $R_3$ et $R_4$, et/ou $R_4$ et $R_5$, forment ensemble, et avec l'atome de carbone auquel ils sont liés, un noyau cyclohexylène,

ainsi que de sels ou de sels complexes métalliques de ces composés avec des acides minéraux ou organiques, procédé caractérisé en ce qu'on fait réagir une amine aromatique répondant à la formule (II):

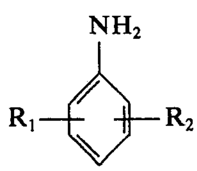

(II)

dans laquelle $R_1$ et $R_2$ ont les significations précédemment données, à température élevée, en présence d'un acide comme catalyseur, dans un milieu aqueux acide contenant au moins 30% en poids d'eau par rapport à l'acide mis en jeu, avec au plus 3 mol d'un agent d'alkylation ou d'aralkylation (III) capable d'introduire un radical Z dans le noyau benzénique de l'amine de formule (II) en position ortho ou en position para relativement au radical amino, et on transforme éventuellement les composés de formule (I), au moyen d'un sel minéral ou organique, en un sel ou en un sel omplexe métallique.

2. Procédé selon la revendication 1, dans lequel le rapport molaire de l'amine de formule (II) à l'agent d'alkylation ou d'aralkylation (III) est compris entre 10 : 1 et 1 : 3.

3. Procédé selon la revendication 1, dans lequel n et $R_1$ ont les significations données à la revendication 1, $R_2$ représente l'hydrogène, $R_3$ l'hydrogène ou un méthyle, $R_4$ un méthyle et $R_5$ un alkyle en $C_1-C_8$ ou un phényle.

4. Procédé selon la revendication 1, dans lequel n a la signification donnée à la revendication 1, $R_1$ représente l'hydrogène ou un halogène, $R_2$ l'hydrogène, $R_3$ l'hydrogène ou un méthyle, $R_4$ un méthyle et $R_5$ un alkyle en $C_1-C_8$ ou un phényle.

5. Procédé selon la revendication 1, caractérisé en ce qu'on effectue la réaction à une température comprise entre 30 et 250° C.

6. Procédé selon la revendication 1, caractérisé en ce qu'on effectue la réaction à une température comprise entre 100 et 190° C.

7. Procédé selon la revendication 1, caractérisé en ce qu'on effectue la réaction à une température supérieure à 110° C et sous une surpression inférieure à 0,987 kPa.

8. Procédé selon la revendication 1, caractérisé en ce qu'on utilise le chlorure d'hydrogène comme catalyseur.

9. Procédé selon la revendication 1, caractérisé en ce qu'on effectue la réaction en présence d'un sel, d'un oxyde, d'un hydroxyde ou d'un carbonate d'un métal, qui joue le rôle de co-catalyseur, le métal étant un de ceux des groupes II, III et VIII de la classification périodique.

10. Procédé selon la revendication 9, caractérisé en ce que le sel métallique est à une concentration au plus égale à 1,0 mol par mole de l'amine aromatique.

11. Procédé selon la revendication 9 dans lequel le co-catalyseur est $ZnCl_2$.

12. Procédé selon la revendication 1 caractérisé en ce qu'on utilise, comme agents d'alkylation ou d'aralkylation (III), des oléfines, des alcools, des alkylamines, des halogénures d'alkyles ou des éthers capables d'introduire le radical Z dans le noyau benzénique de l'amine aromatique de formule (II).

13. Procédé selon la revendication 1, caractérisé en ce qu'on utilise, comme agent d'alkylation, l'isobutène, le di-isobutène, le propène ou l'$\alpha$-méthylstyrène.